# EUROPEAN PATENT SPECIFICATION

(11) **EP 0 437 547 B1**
(45) Date of publication and mention of the grant of the patent: **29.12.1999**
(21) Application number: 90900499.6
(22) Date of filing: 02.10.1989
(51) Int. Cl.: C12N 5/20, C07K 16/00, C07K 16/36, A61K 39/395, G01N 33/536, G01N 33/567

(54) **MONOCLONAL ANTIBODIES AGAINST RECEPTOR-INDUCED BINDING SITES ON LIGANDS BINDING TO PROTEINS OF THE CYTOADHESIN SUPER FAMILY**
MONOKLONALE ANTIKÖRPER GEGEN REZEPTOR-INDUZIERTE BINDUNGSSTELLEN AUF LIGANDEN, DIE AN PROTEINE DER CYTOADHESIN-SUPERFAMILIE BINDEN
ANTICORPS MONOCLONAUX CONTRE DES SITES DE LIAISON INDUITS PAR RECEPTEURS DE LA FAMILLE DES CYTOADHESINES

(30) Priority: 03.10.1988 US 252753
(43) Date of publication of application: 24.07.1991
(73) Proprietor: THE SCRIPPS RESEARCH INSTITUTE, La Jolla, CA 92037 (US)
(72) Inventor: PLOW, Edward, F., San Diego, CA 92122 (US); GINSBERG, Mark, H., San Diego, CA 92122 (US)
(74) Representative: Fisher, Adrian John
(86) International application number: US8904307
(87) International publication number: WO9004634

(56) References cited:
- US-A- 4 722 903
- The Journal of Biological Chemistry, Vol. 263, No. 26, issued 15 September 1988, BENNETT et al., "Interaction of Fibrinogen with its Platelet Receptor", see pages 12948 to 12953.
- Molecular Immunology, Vol. 24, No. 5, issued 1987 (GB), NILSSON et al., "Production of Mouse Monoclonal Antibodies that Detect Distinct Neoantigenic Epitopes on Bound C3b and iC3b but not on the Corresponding Soluble Fragments",
- see pages 487-494.

## Description

### Technical Field

The present invention relates to an antibody binding site that is induced on a ligand when that ligand is bound by a receptor to form a receptor-ligand complex. Monoclonal antibodies that immunoreact with the receptor-induced binding site and therapeutic and diagnostic methods using the monoclonal antibodies are also contemplated.

### Background

Interactions of ligands with cell surface receptor molecules are hallmarks of biological processes. Exemplary of such interactions are the binding of the ligand formed by a portion of the envelope protein (gp140) of the AIDS virus (HIV) with the CD4 receptor on T cells, the receptors of the major histocompatibility complex that interact with numerous ligands in the processes of self/non-self discrimination in the immunological system, and the interaction of the fibrinogen ligand with the GPIIb/IIIa cellular receptor on platelets. The fibrinogen:GPIIb/IIIa ligand-receptor pair are of particular interest in blood clotting, thrombus formation, and are utilized hereinafter as exemplary of ligands and receptors.

The art has long sought immunological methods to distinguish the bound and non-bound forms of ligands and receptors because that ability would allow for a determination of the state of the physiological mechanisms mediated by receptor: ligand complex formation. Until recently, efforts to make such determinations by immunological methods have been frustrated because biological samples can contain ligands and receptors in both bound (complexed) and free forms.

For instance, the vasculature of individuals undergoing a thrombotic event contains non-bound forms of fibrinogen and GPIIb/IIIa as well as platelets having a fibrinogen:GPIIb/IIIa complex on their surface. The fibrinogen:GPIIb/IIIa complex expresses antigenic determinants common to non-bound fibrinogen and non-bound GPIIb/IIIa, thus making it difficult to identify a thrombotic condition or thrombus location by existing immunological methods.

Recently, Frelinger et al., J. Biol. Chem., 263:12397-12402 (1988) reported identifying an antigenic determinant expressed by GPIIb/IIIa only when GPIIb/IIIa was bound to a ligand. That is, the antigenic determinant described by Frelinger et al. was expressed by the receptor of the receptor:ligand complex.

Others have reported the preparation of monoclonal antibodies that immunoreact with an antigenic determinant expressed upon ligand binding to artificial, non-receptor surfaces.

Soria et al., J. Colloid Interface Sci., 107:204-208 (1985) describe a particular monoclonal antibody referred to as DSB² that was induced by immunization with cross-linked fibrin fragment and binds to plastic-adsorbed fibrinogen and also to the so-called fibrinogen D fragment adsorbed on plastic or free in solution, but does not bind to solution phase fibrinogen or the so-called fibrinogen E fragment when in solution.

Nilsson et al., Molec.Immunul., 24:487-94, 1987, describe the preparation of monoclonal antibodies that immunoreact with complement C3 fragments when particle bound to Zymosan A, but not with soluble C3 fragments. The nature of protein binding to Aymosan A involves covalent chemical bonds. Also disclosed are monoclonal antibodies to erythrocyte-bound C3 which do not react with soluble C3.

In another report Abrams et al., Blood (Suppl. 1), 70:355a (Dec. 1987) briefly discuss a monoclonal antibody designated 9F9 that is said in the published abstract to bind to platelet-bound fibrinogen. That brief disclosure, however, did not characterize the specific binding properties of monoclonal 9F9.

Numerous monoclonal antibodies that immunoreact with solubilized fibrinogen have been reported. One such report is Lindon et al., Blood, 68:355-362 (Aug. 1988). That paper reported the use of commercially available anti-human fibrinogen monoclonal antibodies as well as affinity-purified polyclonal antibodies to the D and E fragments.

### Brief Summary of the Invention

It has now been found that ligands that are specifically bound to a receptor can be distinguished from non-bound ligands by the presence of a receptor-induced antibody binding site (RIBS) expressed by the receptor-bound ligand. That is, a class of antigenic determinants has been discovered which are expressed when a ligand specifically binds to a receptor but are not expressed by the non-bound receptor or the non-bound ligand.

A RIBS is expressed on a ligand due to the specific interaction of a ligand binding to its cognate receptor. A RIBS is not an antigenic determinent site that is exposed when a protein interacts non-specifically with another surface, such as plastic-absorbed protein, or when a protein interacts by covalent chemical bonds with a surface. These latter two examples are disclosed in the Soria et al., and Nilsson et al., references described above, and do not involve specific receptor ligand binding interaction that produce a RIBS as disclosed herein.

The present invention relates to antibody molecules that immunoreact with a ligand, preferably fibrinogen, when it is bound to a receptor which is a member of the cytoadhesin super family of proteins, but do no immunoreact with soluble fibrinogen, as for example in plasma.

These antibodies recognize the RIBS induced as a consequence of the fibrinogen interaction of proteins, preferably GPIIb/IIIa, on platelets. The antibodies of this invention act selectively with bound-fibrinogen even with a large excess of plasma fibrinogen. The unique properties of these antibodies therefore allow for a wide variety of diagnostic systems and therapeutic methods of use.

One embodiment of the present invention therefore contemplates a monoclonal antibody that immunoreacts with a receptor-induced binding site expressed by a receptor ligand complex. Preferably the monoclonal antibody is selected from the group consisting of 2G5, 2F10, 3G11, and 4G10.

The monoclonal antibody is produced by a hybridoma selected from the group consisting of hybridoma 2G5, hybridoma 2F10, hybridoma 3G11, and hybridoma 4G10.

Another embodiment of this invention is a cell culture composition which comprises:
a) a hybridoma that produces an antibody that immunoreacts with a receptor-induced binding site expressed by a GPIIb/IIIa:fibrinogen complex, the hybridoma selected from the group consisting of hybridoma 2G5, hybridoma 2F10, hybridoma 3G11 and hybridoma 4G10;
b) antibody molecules secreted by the hybridoma; and
c) a culture medium for the hybridoma.

Further contemplated is a method of detecting the presence of a receptor-ligand complex wherein the system comprises a monoclonal antibody that immunoreacts with a receptor-induced binding site expressed by a receptor-ligand complex, but is not expressed by the non-bound receptor or the non-bound ligand. Preferably the receptor complex is GPIIb/IIIa:fibrinogen. Also preferred is the method wherein the monoclonal antibody is linked to an in vivo indicating means.

Another aspect of this invention is the diagnostic system, in kit form, for the assay of a vascular fluid sample for the presence of the GPIIb/IIIa:fibrinogen complex which system comprises a monoclonal antibody that immunoreacts with a RIBS expressed by this complex.

Another embodiment of this invention is the method of dispersement of a thrombus, in vivo, in a mammal which comprises:
a) intravenously administering to said mammal an effective amount of a monoclonal antibody-plasminogen activating enzyme conjugate wherein the monoclonal antibody of the conjugate immunoreacts with a receptor-induced binding site expressed by a GPIIb/IIIa:fibrinogen complex.

Preferably the plasminogen activating enzyme is tissue plasminogen activator and the monoclonal antibody is selected from the group consisting of 2G5, 2F10, 3G11, and 4G10.

Another use for the in vivo diagnostic and therapeutic abilities of the antibodies of this invention is the method to detect, and locate a thrombus in a mammal which compromises the steps of:
a) intravenously administering to a mammal, an effective amount of a monoclonal antibody that immunoreacts with a receptor-induced binding site expressed by a GPIIb/IIIa:fibrinogen complex;
b) maintaining the administered mammal for a predetermined time period sufficient for the antibody to immunoreact with the complex and form an immunoreaction product; and
c) assaying for the presence of any immunoreaction product formed in step b and thereby the presence of the thrombus.

Preferably the antibody is linked to an in vivo indicating means.

### Detailed Description of the Invention

### A. Definitions

Receptor: Receptor and receptor protein are terms used herein to indicate a biologically active proteinaceous molecule that specifically binds to (or with) other molecules, referred to as ligands, to form a receptor-ligand protein complex.

Ligand and Cognate Ligand: Ligand refers to a molecule that contains a structural portion that is bound by specific interaction with a particular receptor protein.

Receptor-induced Binding Site (RIBS): A RIBS is a neo-antigenic determinant that is expressed by the ligand portion of a receptor-ligand complex but is not expressed by either the non-bound ligand or the non-occupied receptor. A RIBS can be either "conformational" or "sequential". A RIBS is the result of specific alterations of the ligand induced by receptor binding, i.e., a "cryptic antigenic determinant".

Cryptic Antigenic Determinant: Refers to a neo-antigenic determinant formed by changes in conformation of a ligand protein upon binding to its cognate (specific) receptor. Thus, a ligand described herein does not express a cryptic antigenic determinant unless the ligand has specifically bound to a receptor.

"Unit dose" as it pertains to the inocula of the present invention refers to physically discrete units suitable as unitary dosages for animals, each unit containing a predetermined quantity of active material calculated to produce the desired immunogenic effect in association with the required diluent; i.e., carrier, or vehicle. The specifications for the novel unit dose of this invention are dictated by and are directly dependent on (a) the unique characteristics of the active material and the particular immunologic effect to be achieved, and (b) the limitations inherent in the art of compounding such active material for immunologic use in animals, as disclosed in detail herein, these being features of the present invention.

Unit dosage forms are typically prepared from the frozen or dried antibody by dispersement in a physiologically tolerable (acceptable) diluent or vehicle such as water, saline or phosphate-buffered saline to form an aqueous composition. Such diluents are well known in the art and are discussed, for example, in Remington's Pharmaceutical Sciences, 16th Ed., Mack Publishing Company, Easton, PA (1980) at pages 1465-1467.

Dosage forms can also include an adjuvant as part of the diluent. Adjuvants such as complete Freund's adjuvant (CFA), incomplete Freund's adjuvant (IFA) and alum are materials well known in the art, and are available commercially from several sources.

Antigenic Determinant or Antigen: Antigenic determinant or antigen refers to the actual structural portion of the antigen that is immunologically bound by an antibody combining site. The terms are also used interchangeably with epitope.

Neo-antigen: A neo-antigen, as defined herein, is a new antigenic determinant that was not expressed by a ligand prior to binding to the receptor but that is expressed in the ligand-receptor complex.

Antibody: The term antibody in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')₂ and F(v).

Antibody Combining Site: An antibody combining site is that structural portion of an antibody molecule comprised of a heavy and light chain variable and hypervariable regions that specifically binds (immunoreacts with) an antigen. The term immunoreact in its various forms means specific binding between an antigenic determinant-containing molecule and a molecule containing an antibody combining site such as a whole antibody molecule or a portion thereof.

Monoclonal Antibody: The phrase monoclonal antibody in its various grammatical forms refers to a population of antibody molecules that contains only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen, e.g., a bispecific monoclonal antibody.

A monoclonal antibody is typically composed of antibody molecules produced by clones of a single cell such as a hybridoma that secretes (produces) but one kind of antibody molecule. The hybridoma cell is formed by fusing an antibody-producing cell and a myeloma or other self-perpetuating cell line. Such antibodies were first described by Kohler and Milstein, Nature 256:495-497 (1975), which description is incorporated by reference.

### B. Ligand-Receptor Interactions

As noted earlier, ligand-receptor complex formation lies at the heart of many biological processes. Aside from the fact of the existence of such interactions, those interactions lead to subsequent events that are manifest in the biological processes to which complex formation is an initial step.

It has now been found that along with the biological function that accompanies ligand-receptor formation, another, more subtle change occurs. That change is in the conformation of the ligand molecule that results from the binding interaction with the receptor and complex formation. That change in ligand conformation results in the formation of a neo-antigen that is expressed substantially only upon formation of the complex. As noted earlier, that neo-antigen is referred to as a receptor-induced binding site, or RIBS.

The RIBS expressed by the complex formed by the binding of fibrinogen as ligand to GPIIb/IIIa as receptor is used illustratively herein as exemplary of the RIBS-forming phenomenon. Monoclonal antibodies that immunoreact with the fibrinogen-GPIIb/IIIa complex but do not substantially react with either the ligand or receptor when not present in the complex are also utilized herein as exemplary as anti-RIBS (or more simply, RIBS) monoclonal antibodies.

In addition to the exemplary RIBS and RIBS monoclonal antibodies specifically discussed herein, several additional ligand-receptor complexes are reported in the literature. Those complexes also form RIBS and monoclonal antibodies can be raised to those RIBS, using the techniques described herein. Those monoclonal antibodies immunoreact with only the ligand portion of the ligand-bound receptor complex and not with the free receptor or free ligand. Using such RIBS monoclonals, one can now assay for the presence and amount of a ligand-receptor complex; i.e., an occupied receptor or occupied ligand, in the presence of either or both of the free ligand and free receptor.

Exemplary, additional pairs of RIBS-forming ligands and receptors are enumerated in Table 1 that follows. These pairs are also intended to be illustrative and non-limiting of the RIBS that can be formed.

**TABLE 1**

| LIGAND RECEPTOR PAIRS | | |
|---|---|---|
| Ligand | Receptor | Citation No. |
| Von Wildebrand Factor | GPIIb/IIIa | 1 |
| Vitronectin | GPIIb/IIIa | 1 |
| Fibronectin | Fibronectin Receptor | 1 |
| ICAM-1 | LFA-1 | 1 |
| Laminin | CSAT | 2 |
| Collagen | VLA-2 | 3 |
| C3bi | CR3 Complement Receptor | 4 |
| C3d | CR2 Complement Receptor | 5 |
| HIV-gp140 | CD4 T Cell Receptor | 6 |
| FSH Releasing Protein | FRP Receptor | 7 |
| Apo B-100 | Apolipoprotein Receptor | 8 |
| IL-2 | Interleukin Receptor | 9 |
| Immunoglobulins | Fc Receptor | 10 |
| Chorionic Gonadotrophin | Somatostatin Receptor | 11 |
| PDGF | PDGF Receptor | 12 |
| Transferrin | Transferrin Receptor | 13 |
| 1 Ruoslahti et al., Science, 238:491-497 (1987). 2 Horwitz et al., J. Cell. Biol., 101:2134-2144 (1985). 3 Nieuwenhuis et al., Nature, 318:470-472 (1985) 4 Wright et al., Proc. Natl. Acad. Sci. USA, 84:1965-1968 (1987). 5 Nemerow et al J. Virol., 55:347-351 (1985). 6 Guyader et al., Nature, 320:662-669 (1987). 7 Yu et al., Nature, 330:765-67 (1987). 8 Yamada et al., J. Clin. Invest., 80:507 (1987). 9 Dower et al., Immunol. Today, 8:46 (1987). 10 Anderson et al., J. Immunol, 138:2254 (1987). 11 Kim et al., J. Biol. Chem., 262:470 (1987). 12 Keating et al., J. Biol. Chem, 252:7932 (1987). 13 Kohgo et al., Blood, 70:1955 (1987). | | |

### C. Monoclonal Antibodies

A monoclonal antibody (MAb) of the present invention is characterized as comprising antibody molecules that immunoreact with a ligand's receptor-induced binding site.

A receptor-induced binding site (RIBS) is a neo-antigenic determinant that is expressed by a ligand when the ligand is bound by a receptor, but is not expressed by the free (non-bound) ligand. That is, a receptor-ligand complex expresses a RIBS, but the non-occupied receptor and the non-bound ligand do not. A MAb of the present invention immunoreacts with a RIBS, but does not substantially immunoreact with either the unbound (free) ligand or receptor, i.e., when either the ligand or receptor is free in solution, and can therefore distinguish between the receptor-bound and un-bound forms of a ligand because it immunoreacts with the receptor-bound ligand but not with the free ligand. The phrase "does not substantially immunoreact with either the unbound (free) ligand or receptor..." is utilized herein to mean that the monoclonal antibody-(ligand-receptor) immunoreaction is inhibited by no more than about 15 percent, and preferably less, by competitive binding with free ligand or receptors.

In one preferred embodiment, a subject MAb comprises antibody molecules that immunoreact with a RIBS expressed by a cytoadhesin-ligand complex. Cytoadhesin is a name given to a super family of receptor molecules all of which bind to a ligand that contains the amino acid residue sequence arginine-glycine-aspartic acid or RGD. Plow et al., Proc. Natl. Acad. Sci. USA, 83:6002 (1986). This super family has also been given the name Integrin. Rouslahti et al., Science, 238:491-497 (1987). The particular cytoadhesin of exemplary interest herein is GPIIb/IIIa, also known as the platelet receptor.

A preferred monoclonal antibody of this invention is secreted (produced) by each of the following hybridomas: hybridoma 2G5 having ATCC designation HB9847, hybridoma 2F10 having ATCC designation HB9844, hybridoma 3G11 having ATCC designation HB9845, and hybridoma 4G10 having ATCC designation HB9846. The above hybridomas were deposited at the American Type Culture Collection, 12301 Parklawn Drive, Rockville, MD, 20852, USA on September 29, 1988 pursuant to the Budapest Treaty.

### D. Methods for Producing Monoclonal Antibody Compositions

The present invention contemplates a method of forming a monoclonal antibody that immunoreacts with a receptor-induced binding site. The method comprises the steps of:
(a) Immunizing an animal with a receptor-ligand complex. This is typically accomplished by administering an immunologically effective amount, i.e., an amount sufficient to produce an immune response, of immunogen to an immunologically competent mammal. Preferably, the mammal is a rodent such as a rabbit, rat or mouse, although other mammals such as goats, horses, and simians can be used. The mammal is then maintained for a time period sufficient for the mammal to produce cells secreting antibody molecules that immunoreact with the receptor-ligand complex.
(b) A suspension of antibody-secreting cells removed from the immunized mammal is then prepared. This is typically accomplished by removing the spleen of the mammal and mechanically separating the individual spleen cells in a physiologically tolerable medium using methods well known in the art.
(c) The suspended antibody producing cells are treated with a transforming agent capable of producing a transformed ("immortalized") cell line. Transforming agents and their use to produce immortalized cell lines are well known in the art and include DNA viruses such as Epstein Bar Virus (EBV), Simian Virus 40 (SV40), Polyoma Virus and the like, RNA viruses such as Moloney Murine Leukemia Virus (Mo-MuLV), Rous Sarcoma Virus and the like, myeloma cells such as P3X63-Ag8.653, Sp2/O-Ag14 and the like.

In preferred embodiments, treatment with the transforming agent results in the production of a hybridoma by fusing the suspended spleen cells with mouse myeloma cells from a suitable cell line by the use of a suitable fusion promoter. The preferred ratio is about 5 spleen cells per myeloma cell. A total volume of about 10⁸ splenocytes.

The cell line used should preferably be of the so-called "drug resistant" type, so that unfused myeloma cells do not survive in a selective medium, while hybrids will survive. The most common class are 8-azaguanine-resistant cell lines, which lack the enzyme hypoxanthine guanine phosphoribosyl transferase and hence are not supported by HAT (hypoxanthine, aminopterin, and thymidine) medium. It is also generally preferred that the myeloma cell line used be of the so-called "non-secreting" type, in that it does not itself produce any antibody, although secreting types may be used. In certain cases, however, secreting myeloma lines may be preferred. Although the preferred fusion promoter is polyethylene glycol having an average molecule weight from about 1000 to about 4000 (commercially available as PEG 1000, for example), other fusion promoters known in the art may be employed.
(d) The transformed cells are then cloned, preferably to monoclonality. The cloning is preferably performed in a tissue culture medium that will not support non-transformed cells. When the transformed cells are hybridomas, this is typically performed by diluting and culturing in separate containers, the mixture of unfused spleen cells, unfused myeloma cells, and fused cells (hybridomas) in a selective medium that does not support the unfused myeloma cells for a time sufficient to permit death of the unfused cells (about one week). The dilution and culturing is carried out in separate containers, and the dilution may be a limiting dilution in which the volume of diluent is statistically calculated to isolate a certain number of cells (e.g., 1-4) in each separate container (e.g., each well of a microliter plate). The medium is one (e.g., HAT medium) that does not support the drug-resistant (e.g., 8-azaguanine-resistant) unfused myeloma cell line.
(e) The tissue culture medium of the cloned transformants is evaluated for the presence of secreted antibody molecules that do not immunoreact with a free ligand but do immunoreact with the ligand when it is present as part of a receptor-ligand complex. The evaluation is performed using well known immunological techniques, as is described hereinafter.
(f) Once a desired transformant has been identified in step (e), it is selected and grown in a suitable tissue culture medium for a suitable length of time, followed by recovery of the desired antibody from the culture supernatant. The suitable medium and suitable length of culturing time are well known in the art and are readily determined.

To produce a much greater concentration of slightly less pure monoclonal antibody, the desired hybridoma can be injected into mice or other mammals in which the hybridoma can grow, preferably syngeneic or semisyngenic mice. The hybridoma causes formation of antibody-producing tumors after a suitable incubation time, which results in a high concentration of the desired antibody (about 5-20 mg/ml) in the bloodstream and peritoneal exudate (ascites) of the host mouse.

Media useful for the preparation of these compositions are both well known in the art and commercially available, and include synthetic culture media, inbred mice and the like. An exemplary synthetic medium is Dulbecco's minimal essential medium [DMEM; Dulbecco et al., Virol. 8:396 (1959)] supplemented with 4.5 gm/l glucose, 20 mm glutamine, and 20% fetal calf serum. An exemplary inbred mouse strain is the Balb/c.

A monoclonal antibody produced by the above method can be used, for example, in diagnostic and therapeutic modalities, discussed in greater detail hereinafter wherein formation of a RIBS-containing immunoreaction product is desired. Such uses include, for example, is the diagnostic methods and systems of the present invention to detect fibrinogen-bound platelets in a body sample, for in vivo detection, of a thrombus, dispersing a thrombus, or thrombus imaging.

A RIBS monoclonal antibody is typically utilized in an aqueous composition. That composition can be the tissue culture medium or ascites fluid as obtained or in diluted form. Such compositions are typically utilized in vitro.

For in vivo uses, the RIBS monoclonal antibody is typically purified as by precipitation in ammonium sulfate, affinity purification procedure and the like and then utilized in an aqueous composition of a pharmaceutically acceptable diluent. The concentration of RIBS monoclonal antibodies in that aqueous composition is adjusted to suite the desired use.

### E. Therapeutic Methods and Compositions

Monoclonal antibodies secreted by any of the before-described deposited hybridomas, and antibodies having similar immunospecificity, are particularly useful in areas relating to blood clots or thrombi. This is because those types of anti-RIBS monoclonal antibodies immunoreact with the fibrinogen-GPIIb/IIIa receptor complex that is present on activated platelets containing bound fibrinogen, and platelet-bound fibrinogen is involved in thrombus formation. Furthermore, since by definition, those RIBS monoclonal antibodies do not immunoreact with soluble fibrinogen as is present in vivo in circulating blood, extreme specificity of immunoreaction can be had by use of one or more of those monoclonal antibodies.

### 1. Thrombus Dispersment

Thus, one aspect of this invention contemplates a method for the dispersement of a thrombus. Here, the antibody mocules of a monoclonal antibody produced by at least one of the ATCC deposited hybridomas is chemically linked to a plasminogen-activating enzyme to form a conjugate in which the binding of the antibody portion of the conjugate to its RIBS is substantially unimpaired and the plasminogen activating activity of the enzyme is substantially unimpaired. Methods of preparing antibody-protein conjugate in which the activities of both portions of the conjugate are substantially unimpaired are well known by skilled workers.

A plasminogen activating enzyme refers to the group of fibrinolytic drugs in the class of prothrombolysis, such as tissue plasminogen activators that induce thrombolysis without systemic fibrinolysis or fibrinogen break down. Other fibrinolytic drugs contemplated are those which interact with the proactivator plasminogen and include, but are not limited to streptokinase, urokinase (u-PA), and tissue plasminogen activator (t-PA).

In accordance with this method, a pharmaceutically acceptable aqueous composition containing a thrombus-dispersing amount of a before-described conjugate is administered, such as by intravenous injection or infusion, to a mammal such as a human having a thrombus to be dispersed. The mammal so treated (administered mammal) is maintained for a time period sufficient for the antibody portion of the conjugate to immunoreact with the platelet-bound fibrinogen complex present in the thrombus and for the plasminogen-activating enzyme portion of the conjugate to activate plasminogen. Since removal of the conjugate would be a difficult and time-consuming task, the treated mammal is maintained for a time sufficient for its own body to clear the conjugate by usual means.

### 2. Inhibition of Thrombus Formation

Another treatment method is contemplated that is useful for animal subjects that are at risk of thrombus formation such as persons in the first several days after a major operation like a coronary by-pass operation.

Here, a thrombus-inhibiting amount of a monoclonal antibody containing antibody mocules produced by at least one of the ATCC deposited hybridomas 2G5, 2F10, 3G11 or 4C10 present in a pharmaceutically acceptable aqueous composition is administered to a mammal such as a human in whom thrombus formation is to be inhibited. The treated mammal (administered mammal) is maintained for a time period sufficient for the administered RIBS monoclonal antibodies to be cleared from its body by usual means.

In this method, the immunoreaction of the RIBS monoclonal antibodies with activated platelets containing bound fibrinogen inhibits thrombus formation. Of course, since a RIBS monoclonal antibody immunoreacts with the fibrinogen-GPIIb/IIIa complex on the platelet and not with fibrinogen in the blood, normal function of the treated animal is not impaired.

In a related embodiment a method of inhibiting platelet aggregation is contemplated that comprises administering to a platelet containing solution a pharmaceutically acceptable aqueous composition containing monoclonal antibody molecules produced by at least one of the ATCC deposited hybridomas 2G5, 2F10, 3G11 or 4C10. A platelet aggregation-inhibiting amount of the antibody is administered in vivo to a animal subject in which inhibition of platelet aggregation is desired, or is admixed in vitro with a platelet-containing solution, such as plasma or blood. The immunoreaction of the RIBS monoclonal antibodies with fibrinogen-GPIIb/IIIa complex on the platelets forms an immunoreaction product that inhibits platelet aggregation.

A single, before-described, RIBS monoclonal antibody can be used in each of the above methods, or a mixture containing more than one can be utilized. Thus, although each of the monoclonal antibodies produced by the four ATCC deposited hybridomas shares the property of being a RIBS monoclonal, each antibody combining site does not immunoreact with the same epitope. Thus, advantage can be had by using a mixture of those monoclonal antibodies (alone or in the conjugate) so that multiple binding of the combining sites to a single bound fibrinogen molecule can be had.

It is to be understood that although the two above methods have been described in terms of the RIBS monoclonal antibodies that immunoreact specifically with the fibrinogen-GPIIb/IIIa complex, similar methods are applicable for other ligand-receptor complexes that contain RIBS.

The preparation of a pharmaceutically acceptable aqueous composition that contains antibody molecules as active ingredients is well understood in the art. Typically, such compositions are prepared as injectables, either as liquid solutions or suspensions, however, solid forms suitable for solution in, or suspension in, an aqueous liquid prior to injection can also be prepared. The preparation can also be emulsified.

The conjugate or monoclonal antibody alone is often mixed with excipients that are pharmaceutically acceptable and compatible with the conjugate or monoclonal antibody as are well known. Suitable excipients are, for example, water, saline, dextrose, glycerol, ethanol, or the like and combinations thereof. In addition, if desired, the composition can contain minor amounts of auxiliary substances such as wetting or emulsifying agents, pH buffering agents which enhance the effectiveness of the active ingredient.

A conjugate or monoclonal antibody can be formulated into the above aqueous composition as neutralized pharmaceutically acceptable salt forms. Pharmaceutically acceptable salts include the acid addition salts (formed with the free amino groups of the enzyme or antibody molecule) that are formed with inorganic acids such as, for example, hydrochloric or phosphoric acids, or such organic acids as acetic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, 2-ethylaminoethanol, histidine, procaine, and the like.

The therapeutic antibody molecule-containing compositions are conventionally administered intravenously, as by injection of a unit dose, for example. A composition is administered in a manner compatible with the dosage formulation, and in a therapeutically effective, i.e., in a thrombus-dispersing or thrombus-inhibiting, amount.

The quantity to be administered depends, inter alia, on the animal species to be treated, the subject animal's size, the size of the thrombus (if known), the amount of fibrinogen-bound platelets present, and capacity of the subject to utilize the conjugate or monoclonal antibody. Precise amounts of conjugate or monoclonal antibody required to be administered depend on the judgment of the practitioner and are peculiar to each individual, particularly where humans are the treated animals. Dosage ranges, however, can be characterized by a therapeutically effective blood concentration and can range from a concentration of antibody-containing conjugate or antibody alone of the present invention from about 0.01 uM to about 100 uM, preferably about 0.1 uM to lOuM.

Suitable regimes for initial administration and booster injections are also variable, but are typified by an initial administration followed by repeated doses at one or more hour intervals by a subsequent injection or other administration. Alternatively, continuous intravenous infusion sufficient to maintain therapeutically effective concentrations in the blood are contemplated.

### F. Diagnostic Systems

A diagnostic system in kit form of the present invention includes, in an amount sufficient for at least one assay, a RIBS monoclonal antibodies of the present invention, such as one produced by one of the four ATCC deposited hybridomas, as a separately packaged reagent. A label for indicating the presence of an immunoreaction between the RIBS and RIBS monoclonal antibody is also preferably included in the same or a separate package. Instructions for use of the packaged reagent are also typically included.

Instructions for use typically include a tangible expression describing the reagent concentration or at least one assay method parameter such as the relative amounts of reagent and sample to be admixed, maintenance time periods for reagent/sample admixtures, temperature, buffer conditions and the like.

One embodiment of this invention is a diagnostic system in kit form for assaying for fibrinogen-bound platelets in a platelet-containing vascular fluid sample, such as blood or plasma. The system comprises a package containing a monoclonal antibody that immunoreacts with a RIBS expressed by the receptor-ligand complex. Preferably, the anti-RIBS antibody molecules of the monoclonal antibody are those produced by one of the following hybridomas: hybridoma 2G5, hybridoma 2F10, hybridoma 3G11, and hybridoma 4G10. Preferably, the antibody molecules are present as a monoclonal antibody composition which contains more than one particular monoclonal antibody. Further preferred are kits wherein the antibody molecules are linked to a radionuclide label, preferably a ¹²⁵I-labeled or other labeled antibody molecules. Useful labels are discussed hereinafter.

In another embodiment, a diagnostic system of the present invention is suitable for assaying for the presence of a thrombus in vivo. The system comprises a package containing monoclonal antibody molecules that immunoreact with a RIBS expressed by a receptor-ligand complex. Preferably, the antibody molecules present are those secreted by a hybridoma selected from the group consisting of 2G5, 2F10, 3G11, and 4G10. The antibody molecules preferably linked to an in vivo label or indicating means.

Although a kit for in vivo imaging can often be utilized for in vitro assays, it is understood that the converse need not be true. For example, the monoclonal antibodies utilized for in vivo work should be free of pyrogens as should any buffer salts of aqueous compositions and reagents. Freedom from pyrogen content is not a necessity for in vitro assays. Additionally, the indicating means useful for in vivo imaging is typically different from that used in vitro, as is discussed hereinafter. Thus, the assay system is "suitable" for in vivo imaging, and "suitable" buffer salts, aqueous solutions and indicating means can be supplied as part of the kit in the same or separate packages.

In preferred embodiments, a diagnostic system of the present invention further includes a label or indicating means capable of signaling the formation of a complex containing an antibody molecule of the present invention.

As used herein, the terms label and indicating means in their various grammatical forms refer to single atoms and molecules that are either directly or indirectly involved in the production of a detectable signal to indicate the presence of a complex. In vivo labels or indicating means are those useful within the body of a human subject and include ¹¹¹In, ⁹⁹Tc, ⁶⁷Ga, ¹⁸⁶Re, ¹³²I, and ¹¹¹indium.

Any label or indicating means can be linked to or incorporated in an antibody molecule that is part of a conjugate or monoclonal antibody composition of the present invention, and those atoms or molecules can be used alone or in conjunction with additional reagents. Such labels are themselves well known in clinical diagnostic chemistry and constitute a part of this invention only insofar as they are utilized with otherwise novel protein methods and/or systems.

The linking of labels, i.e., labeling of, to antibodies is well known in the art. For instance, antibody molecules produced by a hybridoma can be labeled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas, et al., Scand. J. Immunol., Vol. 8 Suppl. 7:7-23 (1978), Rodwell et al., Biotech., 3:889-894 (1984), and U.S. Pat. No. 4,493,795.

The in vitro diagnostic systems can also include, preferably as a separate package, a specific binding agent. A "specific binding agent" is a molecular entity capable of selectively binding a monoclonal antibody of the present invention but is not itself an antibody molecule of the present invention. Exemplary specific binding agents are second antibody molecules, complement proteins or fragments thereof, S. aureus protein A and the like. The specific binding agent binds the RIBS monoclonal antibody molecule of this invention when that monoclonal antibody is present as part of an immunocomplex with the ligand-receptor complex. In preferred embodiments, the specific binding agent is labeled. However, when the diagnostic system includes a specific binding agent that is not labeled, the specific binding agent is typically used as an amplifying means or reagent and a second reagent that is labeled binds to the specific binding agent (amplifying means). In these embodiments, the labeled second reagent is capable of specifically binding the amplifying means when the amplifying means is bound to a RIBS monoclonal antibody-containing immunocomplex.

The diagnostic kits of the present invention can be used in an "ELISA" format to detect the presence or quantity of ligand-receptor complex such as fibrinogen-bound platelets in a body fluid sample such as serum, plasma or urine. "ELISA" refers to an enzyme-linked immunosorbent assay that employs an antibody or antigen bound (here, the RIBS-containing ligand-receptor complex) to a solid phase matrix that forms a solid support and an enzyme-antigen or enzyme-antibody conjugate to detect and quantify the amount of an antigen or antibody present in a sample. A description of the ELISA technique is found in Chapter 22 of the 4th Edition of Basic and Clinical Immunology by D.P. Sites et al., published by Lange Medical Publications of Los Altos, CA in 1982 and in U.S. Patents No. 3,654,090; No. 3,850,752; and No. 4,016,043, which are all incorporated herein by reference.

In preferred ELISA kit embodiments, the antibody molecules of the present invention are affixed to a solid matrix to form a solid support that is separately packaged in the subject diagnostic systems. The antibodies are typically affixed to the solid matrix by adsorption from an aqueous medium although other modes of affixation, well known to those skilled in the art can be used.

A labeled specific binding agent that binds to the complex or to its constituents or an unlabeled specific binding agent plus a labeled second reagent that binds to the specific binding agent is also included in one or two separate packages, respectively, in the kit. Using one of the monoclonal antibodies produced by one of the ATCC deposited hybridomas as exemplary of the matrix-bound antibody, labeled anti-fibrinogen antibodies as are commercially available are exemplary of the specific binding agent.

Useful solid matrices are well known in the art. Such materials include the cross-linked dextran available under the trademark SEPHADEX from Pharmacia Fine Chemicals (Piscataway, NJ); agarose; beads of polystyrene beads about 1 micron to about 5 millimeters in diameter available from Abbott Laboratories of North Chicago, IL; polyvinyl chloride, polystyrene, cross-linked polyacrylamide, nitrocellulose- or nylon-based webs such as sheets, strips or paddles; or tubes, plates or the wells of a microliter plate such as those made from polystyrene or polyvinylchloride.

The monoclonal antibodies (labeled or unlabeled), labeled specific binding agent or amplifying reagent of any diagnostic system described herein can be provided in solution, as a liquid dispersion or as a substantially dry power, e.g., in lyophilized form. Where the indicating means is an enzyme, the enzyme's substrate can also be provided in a separate package of a kit system. A solid support matrix such as the before-described microliter plate and one or more buffers can also be included as separately packaged elements in this diagnostic assay system.

The packages discussed herein in relation to diagnostic systems are those customarily utilized in diagnostic systems. Such packages include glass and plastic (e.g., polyethylene, polypropylene and polycarbonate) bottles, vials, plastic and plastic-foil laminated envelopes and the like.

### G. Assay Methods

The present invention contemplates a method for detecting a receptor-ligand complex, as, for example, is found in a thrombus or in fibrinogen-bound platelets, preferably, GPIIb/IIIa:fibrinogen. The method utilizes the expression of a receptor-induced binding site (RIBS) and a monoclonal antibody molecule that immunoreacts with the RIBS in the ligand portion of the receptor-ligand complex, but does not react with a non-bound receptor or a non-bound ligand. Those skilled in the art will understand that there are numerous well known clinical diagnostic chemistry procedures that can be utilized to form those immunocomplexes. Thus, while exemplary assay methods are described herein, the invention is not so limited.

### 1. Thrombus Detection

More specifically, a method for detecting the presence of a thrombus in a mammal such as a human is contemplated. An aqueous composition containing an imaging-effective amount of a monoclonal antibody of the present invention containing antibody molecules linked to an in vivo indicating means is intravenously administered to a mammal such as a human in need of such treatment.

The administered mammal is maintained for a predetermined time period sufficient for the labeled antibody molecule to immunoreact with the platelet-bound fibrinogen complex present as part of a thrombus. The subject mammal is then assayed for the presence and preferably location of any labeled immunocomplex formed, and thereby detects the thrombus and is location. Since the labeled RIBS monoclonal antibodies normally contain radionuclides as the label or indicating means, the imaging assay is carried out by usual, well known radioimaging techniques. Such techniques can distinguish the relatively high concentration of radiolabel at the thrombus from the relatively lower systemic amount of radio label. Where relatively long-lived radioisotope are utilized, the administered mammal can be maintained for a time period sufficient for substantial systemic clearance of the labeled monoclonal antibody to thereby provide a relatively still lower amount of background signal from the radiolabel.

### 2. Detection of Fibrinogen-Bound Platelets in a Body Sample

Various heterogeneous and homogeneous assay protocols can be employed, either competitive or non-competitive, for detecting the presence and preferably amount of fibrinogen-bound platelets in a platelet-containing and/or free fibrinogen-containing body sample, preferably a body fluid sample such as blood or a platelet-containing portion of blood. For example, a heparin-preserved (non-clotted) blood sample and an ¹²⁵I-labeled form of one of the before-discussed, deposited RIBS antibody molecules are admixed. Amounts and concentrations of sample and labeled monoclonal antibody are, of course, utilized so that a meaningful result can be obtained. The immunoreaction admixture thus formed is maintained under biological assay conditions for a time period sufficient for fibrinogen-bound platelets present in the sample to immunoreact with the labeled antibodies and form a labeled immunoreaction product. The labeled immunoreaction product, when present, is then separated from any non-reacted labeled-antibodies that may be present. In homogenous assays, separation is typically by centrifugation sufficient to pellet all platelets present in the sample. In heterogeneous assays such as an ELISA, the immunoreaction product is bound to the solid support and the separation is typically effected by a washing step in which any unbound RIBS antibody is discarded and the solid support-bound immunoreaction product is retained.

It should be understood that where an unlabeled RIBS monoclonal antibody is used to immunoreact with a RIBS-containing ligand-receptor complex, a second admixture is formed between the before-described, separated immunocomplex and a labeled binding reagent or unlabeled binding reagent used as an amplifying means. That reaction mixture is maintained under biological conditions for a time period sufficient for a second binding complex to form between the first-formed immunocomplex and the specific binding reagent. (Where the specific biding reagent comprises antibody molecules, that second binding complex is a second immunocomplex. Where S. aureus protein A is used, for example, the second binding complex does not rely for binding upon the antibody binding site, and that complex is best referred to as a binding complex. Since an immunocomplex is a specific binding complex, the complex formed between the specific binding reagent and first-named immunocomplex is a second binding complex.) The second binding complex is thereafter separated from any unreacted specific binding reagent that may be present, as by a previously mentioned technique, and the presence of the label and thereby immunocomplex is determined, and preferably quantified.

Where the specific binding reagent is not labeled and is used as an amplifying means, a third admixture is formed from the above, separated second biding complex, when present, and a label-containing second binding reagent. The presence of the label in the second binding complex is, of course, not determined in the above-recited method where no labeled reagent was admixed. The before-described maintenance and separation steps are repeated for this aspect of the method with a label-containing third binding complex being formed and retained. The presence and preferably the amount of the label is thereafter determined.

Thus, in each of the above-described aspects of this assay, the presence and preferably the amount of the label provides the basis for determining the presence and preferably the amount of fibrinogen-bound platelets.

It is noted that the worker carrying out an above-described assay usually will not know whether one or more of the immunocomplex or binding complexes has indeed formed until the amount of label present is determined. Nevertheless, all of the steps of a given assay procedure are carried out as if the RIBS-containing complex were indeed present.

It is to be emphasized that because of the unique specificity of the RIBS monoclonal antibodies, the above-described assay for fibrinogen-bound platelets and the before-described imaging assay for a thrombus can be and preferably are carried out in the presence of both free platelets and free fibrinogen, i.e., non-complexed platelets and fibrinogen. As a result, special handing procedure such as separations and washing steps need not be carried out on the body sample prior to use of that sample in an assay. This feature is common to all assays using RIBS monoclonal antibodies.

Biological assay conditions are those that maintain the biological activity of the antibody molecules of this invention and the fibrinogen-bound platelets or other RIBS-containing complex sought to be assayed. Those conditions include a temperature range of about 4 degrees C to about 45 degrees C, preferably about 37 degrees C, a pH value range of about 5 to about 9, preferably about 7 and an ionic strength varying from that of distilled water to that of about one molar sodium chloride, preferably about that of physiological saline. Methods for optimizing such conditions are well known in the art.

### EXAMPLES

The following examples are intended to illustrate, but not limit, the present invention.

### 1. Hybridoma and Monoclonal Antibody Production

Monoclonal antibodies were produced using standard hybridoma technology. Briefly, Balb/c mice were immunized and subsequently boosted three times with about 50 micrograms (ug) per mouse of fibrin D-dimer immunogen prepared substantially as described in Cierniewski et al., Thromb. Haemostas., 48:33-37 (1982).

Subsequently, 1.23 X 10⁸ splenocyte cells from one immunized mouse whose antibodies immunoreacted with the immunogen were admixed with 2.46 X 10⁷ P3Ag8653.1 mouse myeloma cells in the presence of the cell fusion promoter polyethylene glycol 4000. The antibody producing cells thus transformed were transferred to 96-well microtiter plates at a density of about 3 X 10⁴ cells per well and cultured.

Tissue culture supernatants from 235 wells appearing to contain viable hybridomas after about 14 days of culturing were screened by radioimmune assay for the presence of anti-RIBS antibody molecules. Briefly, 100 microliters (ul) of phosphate-buffered saline (PBS) containing either 1 ug/ml of fibrinogen or low density lipoprotein (LDL) (control) were admixed into the wells of flat-bottom 96-well polyvinyl microtiter plates as solid phase matrix. The plates were then maintained for about 16-20 hours at 4°C to permit the fibrinogen or LDL to adsorb onto the surface of the wells to form a solid support. The coating solution was removed by shaking, the wells were rinsed, and 100 ul of blocking solution (PBS containing 5% normal goat serum) were admixed into each well to block excess protein binding sites.

The wells were maintained for about 30 minutes at 37°C and then the blocking solution was removed. Into each well were then admixed 100 ul of either (a) hybridoma tissue culture supernatant diluted 1:10 in PBS, or (b) hybridoma supernatant diluted 1:10 in PBS containing 100 ug/ml fibrinogen as a competitive inhibitor. The immunoreactions admixtures thus formed were maintained at room temperature for about 16-20 hours at 4°C to permit the formation of a solid phase-bound immunoreaction product and a liquid phase, including any non-bound monoclonal antibody molecules.

To each well were then admixed 100 ul of ¹²⁵I-labeled goat anti-mouse IgG. The labeling immunoreaction admixture thus formed was maintained about 6-20 hours at 4°C to permit formation of a ¹²⁵I-labeled second solid-phase immunoreaction product. The solid and liquid phases were separated to remove any non-bound ¹²⁵I-goat anti-mouse IgG. The amount of ¹²⁵I-bound to each well was determined by gamma scintillation.

The presence of at least about 3 times the amount of non-specifically bound ¹²⁵I, as determined from the LDL-coated wells, in a fibrinogen-coated well indicated the presence of anti-fibrinogen antibodies in a tissue culture supernatant. A reduction of solid-phase bound ¹²⁵I by no more than about 15% by the presence of liquid-phase fibrinogen competitor in the immunoreaction admixture [part (b) above] indicated the presence of an anti-RIBS antibodies in the tissue culture supernatant.

The above screening procedure resulted in the identification of 4 hybridomas, designated 2G5, 2F10, 3G11 and 4G10, that produce anti-RIBS antibodies that bind a fibrinogen:GPIIb/IIIa complex.

Each of the four above described hybridomas was cloned twice by limiting dilution and subsequently used to produce ascites fluid. The antibodies were then isolated from the ascites fluids by using protein A Sepharose.

Compositions containing Fab fragments of the monoclonal antibody (Mab) 2F10 were prepared by digestion of protein A Sepharose - isolated Mab 2F10 with papain (200:1 weight per weight of Mab to papain) for 6 hours at 37C following the methods of Mage et al., Methods in Enzymology, 70: 142-50 (1980). Undigested antibody and Fc fragments were removed from the Fab fragments by chromatoraphy on protein A Sepharose. The resulting Fab fragments were collected from the Separose to form a 2F10 Fab preparation.

### 2. Detection of a Fibrinogen:GPIIb/IIIa RIBS on Activated Platelets

Monoclonal antibodies produced by hybridomas 2G5, 2F10 and 3G11, i.e. MAb 2G5, MAb 2F10 and MAb 3G11, respectively, were examined for their ability to immunoreact with a cell surface bound RIBS. Each of the four monoclonals was ¹²⁵I labeled using standard Chloramine-T methodology. Greenwood et al., Bio. Chem. J., 89:114-123 (1963).

Sixty milliliters (ml) of whole human blood was collected in 5 ml of ACD (0.065 M citric acid, 0.085 M sodium citrate, 2% dextrose) containing hirudin (Sigma Chemical Co., St. Louis, MO) at a formula concentration of 0.06 units per ml (U/ml) and centrifuged for 15 minutes at 120 x g. The resulting supernant, designated platelet rich plasma, was recovered, isolated and further centrifuged for 15 minutes at 1200 x g to form a pellet of isolated platelets.

The isolated platelets were resuspended in 2 ml of calcuim-free Tyrodes buffer (0.13 M NaCl, 0.0026 M KCI, 0.002 M MgCl₂-6H₂O, 5mM Hepes, 0.012 M NaHCO₃, pH7.2) containing 1 mg/ml bovine serum albumin (BSA) and 1 mg/ml dextrose. The platelet suspension was then applied to a Sepharose CL2B column (40 ml total bed volume; Pharmacia Inc., Piscataway, NJ) equilibrated with the same Tyrodes' buffer. Washed platelets were recovered from the void volume of the CL2B column in a final volume of about 4 to 5 ml.

Samples of the washed platelets were then stimulated (activated) by admixture with either adenosine diphosphate (ADP) to concentration of 10 micromolar (uM), or thrombin to a concentration of 0.1 units/ml. Some samples of ADP-stimulated platelets were also admixed with fibrinogen to a fibrinogen concentration of 1 mM.

To each sample of platelets, including some non-simulated control samples, was admixed a ¹²⁵I-labeled MAb to a concentration of 10 nanomolar (nM). The immunoreaction admixture thus formed was maintained for 30 minutes at 22°C to permit immunoreaction product formation. The immunoreaction products were separated from non-bound ¹²⁵I-MAb by centrifugation through 0.3 ml of 20% sucrose. The amount of ¹²⁵I-MAb associated with the platelet pellet was determined by scintillation spectrometry.

The results of this study, shown in Table 1, indicate that the anti-RIBS monoclonal antibodies do not substantially immunoreact with non-stimulated platelets. However, when the platelets are stimulated with an agonist such as ADP or thrombin, a significant immunoreaction of the MAbs with the fibrinogen:GPIIb/IIIa complex on the cells is obtained. Stimulation of the platelets with ADP or thrombin results in secretion and surface binding by GPIIb/IIIa of the platelet-endogenous fibrinogen. Addition of exogenous fibrinogen did not neutralize (inhibit) the binding of ¹²⁵I-MAb to the stimulated platelets, thus indicating the MAbs are RIBS-specific, i.e., they do not immunoreact with free fibrinogen in solution. Similar results were obtained with MAb 4G10.

**TABLE 1**

| | ¹²⁵I-MAb Bound | | |
|---|---|---|---|
| | (cpm/platelet) | | |
| Platelets | 2G5 | 2F10 | 3G11 |
| Nonstimulated | 1,200 | 2,800 | 1,300 |
| ADP-stimulated | 33,750 | 43,600 | 31,550 |
| ADP-stimulated + fibrinogen | 35,600 | 51,300 | 30,150 |
| Thrombin-stimulated | 28,620 | 32,000 | 28,400 |

To emphasize the point that exogenously added fibrinogen is not cell-associated (i.e., is not part of cell receptor-ligand complex) but yet does not neutralize the immunoreactivity of MAbs 2G5, 2F10, 3G11 and 4G10 with the fibrinogen:GPIIb/IIIa complex, the immunoreactivity of the MAbs with the platelets was examined using platelet rich plasma containing 2-3 mg/ml fibrinogen.

As shown in Table 2, despite the vast excess of free fibrinogen, each of the four ¹²⁵I-MAbs immunoreacted with fibrinogen:GPIIb/IIIa RIBs on the platelet surface.

**TABLE 2**

| | ¹²⁵I-MAb Bound | | | |
|---|---|---|---|---|
| | (cpm/platelet) | | | |
| Platelets | 2G5 | 2F10 | 3G11 | 4G10 |
| Nonstimulated | 174 | 662 | 218 | 678 |
| ADP-stimulated | | 17,823 | 7,471 | 20,38219,540 |

As a further indication of specificity of the four deposited Mabs for RIBS, similar Mab binding studies were conducted using cells containg a Mac-1 receptor rather than platelets having the GPIIb/IIIa platelet glycoprotein receptor. Both Mac-1 and GPIIb/IIIa bind specifically to fibrinogen in a receptor-ligand fashion, but the two interactions produce distinct biological results. In the binding studies, the four deposited RIBS do not exhibit immunoreaction with fibrinogen-Mac-1 complex, but do immunoreact with fibrinogen in a fibrinogen-GPIIb/IIIa complex. Therefore, the four Mabs tested immunoreact specifically with RIBS on fibrinogen expressed incomplex with GPIIb/IIIa but not with RIBS on fibrinogen when complexed with Mac-1.

### 3. Inhibition of Platelet Aggregation by RIBS Antibodies

Two hundred ul of isolated platelets prepared as described in Example 2 were admixed with 190 ul Tyrode's buffer containg BSA and dextrose (each at 1 mg/ml), fibrinogen (1mM), calcium (5mM), and a Fab fragment of Mab 2F10, prepared in Example 2 and present in varying amounts as indicated in Table 3. Ten ul of ADP (80 uM in Tyrode's buffer) were then admixed to stimulate platelet aggregation. The admixture was maintained at 37 degrees C while changes in light transmission of the admixture were monitored over time using a Dual Sample Aggregation Meter (Model DP-247E, Sienco Inc., Morrison, CO).

The aggregation meter was calibrated using a solution containg 200 ul PRP and 200 ul Tyrode's buffer to set a low baseline of light transmission at 5 percent for control aggregations and at 10 percent for aggregations in the presence of antibody. The upper limit of 100 ml PRP and 300 ul Tyrode's buffer.

The results obtained when measuring platelet aggregation inhibition by antibody are shown in Table 3, and are expressed as a percent of light transmission (100%) obtained in the absence of antibody when measured about 3 to 4 minutes after ADP was admixed.

**TABLE 3**

| INHIBITION OF PLATELET AGGREGATION BY MONOCLONAL ANTIBODY 2F10 | |
|---|---|
| [FAB] | Percent Transmission |
| 0 uM | 100 |
| 0.25 uM | 79 |
| 0.5 uM | 62.5 |
| 1.25 uM | 34.5 |
| 1.87 uM | 17.5 |

The results in Table 3 show that the Mab 2F10 fragment produced a dose-dependent inhibition of platelet aggregation. Thus, the results indicate the effective dosages useful to inhibit platelet aggregation and processes involving platelet aggregation, such as thrombus formation, when using antibodies of the present invention that immunoreact with a RIBS specific for fibrinogen: GPIIb/IIIa complex.

The foregoing specification, including the specific embodiment, is intended to be illustrative of the present invention and is not to be taken as limiting. Numerous other variations and modifications can be effected without departing from the scope of this invention, as defined in the claims.

## Claims

1. A monoclonal antibody that immunoreacts with a receptor-induced binding site expressed by a ligand when said ligand is present in a receptor-ligand complex, but does not immunoreact with said ligand or with said receptor when either said ligand or said receptor is free in solution, the receptor of said complex being a member of the cytoadhesin super family of proteins.

2. The monoclonal antibody of claim 1 wherein said receptor is GPIIb/IIIa.

3. The monoclonal antibody of claim 2 wherein said ligand is fibrinogen.

4. The monoclonal antibody of claim 3 wherein said antibody is secreted by a hybridoma selected from the group consisting of hybridoma 2G5, hybridoma 2F10, hybridoma 3G11 and hybridoma 4G10, having ATCC designations HB9847, HB9844, HB9845 and HB9846, respectively.

5. A hybridoma that secretes a monoclonal antibody according to any preceding claim.

6. The hybridoma of claim 5 wherein said hybridoma is selected from the group consisting of hybridoma 2G5, hybridoma 2F10, hybridoma 3G11 and hybridoma 4G10, having ATCC designations HB9847, HB9844, HB9845 and HB9846, respectively.

7. A cell culture composition comprising:
a) a hybridoma according to claim 5 or claim 6;
b) antibody molecules secreted by said hybridoma; and
c) a culture medium for said hybridoma.

8. A diagnostic system in kit form comprising monoclonal antibodies according to any of claims 1 to 4, said monoclonal antibodies being present in a package in an amount sufficient to perform at least one assay.

9. The diagnostic system of claim 8 wherein said monoclonal antibodies are linked to an indicating means.

10. The diagnostic system of claim 9 wherein said indicating means is packaged separately from said monoclonal antibodies.

11. The diagnostic system of claim 9 wherein said indicating means is linked to said monoclonal antibodies and is an in vivo indicating means.

12. An aqueous composition for intravenous administration to disperse a thrombus in a mammal, said composition containing a pharmaceutically acceptable diluent and an amount of a monoclonal antibody-plasminogen-activating enzyme conjugate effective to disperse said thrombus, wherein said monoclonal antibody portion of said conjugate is a monoclonal antibody according to claim 3.

13. The composition of claim 12 wherein said plasminogen-activating enzyme portion of said complex is selected from the group consisting of streptokinase, urokinase and tissue plasminogen activator.

14. The composition of claim 13 wherein said monoclonal antibody portion of said conjugate is secreted by a hybridoma selected from the group consisting of hybridoma 2G5, hybridoma 2F10, hybridoma 3G11 and hybridoma 4G10, having ATCC designations HB9847, HB9844, HB9845 and HB9846, respectively.

15. An aqueous composition for detecting a thrombus in a patient, said composition containing a pharmaceutically acceptable diluent and an imaging-effective amount of a monoclonal antibody according to claim 3 or claim 4 linked to an in vivo radioisotope label.

16. An antibody composition comprising at least two monoclonal antibodies secreted by a hybridoma selected from the group consisting of hybridoma 2G5, hybridoma 2F10, hybridoma 3G11 and hybridoma 4G10, having ATCC designations HB9847, HB9844, HB9845 and HB9846, respectively.

17. An aqueous composition for inhibiting thrombus formation in a patient, said composition containing a pharmaceutically acceptable diluent and a monoclonal antibody according to claim 3 or claim 4.

18. An aqueous composition for inhibiting platelet aggregation in a patient, said composition containing a pharmaceutically acceptable diluent and a monoclonal antibody according to claim 3 or claim 4.

19. A method of forming a monoclonal antibody molecule that immunoreacts with a receptor induced binding site expressed by a ligand-containing receptor-ligand complex wherein said complex contains a cell surface receptor specifically bound to a ligand, which method comprises:
a) immunizing a mammal with a composition containing said complex;
b) removing antibody-producing cells from said immunized mammal and preparing a suspension of said cells;
c) treating said cells with a transforming agent to produce transformed antibody-producing cells;
d) cloning the cells treated in step c) by a limiting dilution in a tissue culture medium that will not sustain non-transformed cells, to produce cloned transformants;
e) assaying the tissue culture medium of the cloned transformants to detect the presence of secreted antibody molecules that immunoreact with a receptor-induced binding site present on the ligand in said receptor-ligand complex but do not immunoreact with the cell surface receptor or the ligand when either is in non-bound form thereby identifying a cloned transformant producing said antibody molecules;
f) growing said identified cloned transformant in tissue culture medium under conditions for producing said secreted antibody molecules; and
g) harvesting said secreted antibody molecules from the culture medium of step (f),
wherein said ligand is fibrinogen and said receptor is the GPIIb/IIIa platelet glycoprotein receptor.

20. An in vitro method of assaying for the presence of a receptor-ligand complex in a vascular fluid sample wherein said complex contains a cell surface receptor specifically bound to a ligand, which method comprises the steps of:
a) forming an immunoreaction admixture by admixing a vascular fluid sample with a monoclonal antibody composition containing antibody molecules that immunoreact with a receptor-induced binding site on the ligand in said receptor-ligand complex but do not immunoreact with the cell surface receptor or the ligand when either is in non-bound form;
b) maintaining the admixture for a time period sufficient for said antibodies to immunoreact with any receptor-ligand complex present in the sample and form an immunoreaction product; and
c) detecting the presence of any immunoreaction product formed in step (b) and thereby the presence of said complex in said sample,
wherein said ligand is fibrinogen and said receptor is the platelet glycoprotein GPIIb/IIIa.

21. The method of claim 20 wherein said antibody molecules are the antibody molecules produced by a hybridoma selected from the group consisting of hybridoma 2G5, hybridoma 2F10, hybridoma 3G11 and hybridoma 4G10, having ATCC designations HB9847, HB9844, HB9845 and HB9846, respectively.

## Patentansprüche

1. Monoklonaler Antikörper, der mit einer Rezeptor-induzierten Bindestelle eine Immunoreaktion eingeht, die durch einen Liganden exprimiert wird, wenn der Ligand in einem Rezeptor-Liganden Komplex vorliegt, der aber mit diesem Liganden oder mit dem Rezeptor keine Immunoreaktion eingeht, wenn entweder der Ligand oder der Rezeptor frei in Lösung vorliegt, wobei der Rezeptor des Komplexes ein Mitglied der Zytoadhäsin-Proteinsuperfamilie ist.

2. Der monoklonale Antikörper nach Anspruch 1, wonn der Rezeptor GPllb/llla ist.

3. Der monoklonale Antikörper nach Anspruch 2, worin der Ligand Fibnnogen ist.

4. Der monoklonale Antikörper nach Anspruch 3, wobei der Antikörper durch ein Hybridom sezemiert wird, ausgewählt aus der Gruppe, bestehend aus dem Hybndom 2G5, dem Hybndom 2F10, dem Hybridom 3G11 und dem Hybridom 4G10, mit den ATCC Bezeichnungen HB9847, HB9844, HB 9845 bzw. HB 9846.

5. Hybndom, das einen monoklonalen Antikörper nach einem der vorstehenden Ansprüche sezemiert.

6. Hybndom nach Anspruch 5, wobei das Hybridom aus der Gruppe ausgewählt ist, bestehend aus dem Hybridom 2G5, dem Hybndom 2F10, dem Hybndom 3G11 und dem Hybndom 4G10, mit den ATCC Bezeichnungen HB9847, HB9844, HB9845 bzw. HB9846.

7. Zellkulturzusammensetzung, umfassend:
a) ein Hybridom nach Anspruch 5 oder 6;
b) Antikörper-Moleküle, sezemiert durch das Hybndom; und
c) ein Kulturmedium für das Hybndom.

8. Diagnostiksystem in Kitform, umfassend monoklonale Antikörper nach einem oder mehreren der Ansprüche 1 bis 4, wobei die monoklonalen Antikörper in einer Packung in einer ausreichenden Menge vorliegen um mindestens einen Assay durchzuführen.

9. Diagnostisches System nach Anspruch 8, wobei die monoklonalen Antikörper mit einem Indikationsmittel verbunden sind.

10. Diagnostisches System nach Anspruch 9, wobei das Indikationsmittel separat von den monoklonalen Antikörpem verpackt ist.

11. Diagnostisches System nach Anspruch 9, wobei das Indikationsmittel mit den monoklonalen Antikörpern verbunden ist und ein in vivo Indikationsmittel ist.

12. Wässrige Zusammensetzung zur intravenösen Verabreichung um einen Thrombus in einem Säugetier aufzulösen, wobei die Zusammensetzung ein pharmazeutisch akzeptables Verdünnungsmittel und eine Menge eines monoklonalen Antikörper-Plasminogen-aktivierenden Enzym-Konjugats enthält, das wirksam ist um den Thrombus aufzulösen, wobei der monoklonale Antikörper-Anteil des Konjugats ein monoklonaler Antikörper nach Anspruch 3 ist.

13. Zusammensetzung nach Anspruch 12, wobei der Plasminogen-aktivierende Enzymanteil des Komplexes ausgewählt ist aus der Gruppe, bestehend aus Streptokinase, Urokinase und Gewebeplasminogen-Aktivator.

14. Zusammensetzung nach Anspruch 13, wobei der monoklonale Antikörper-Anteil des Konjugats durch ein Hybridom sezeniert wird, ausgewählt aus der Gruppe, bestehend aus dem Hybridom 2G5, dem Hybridom 2F10, dem Hybridom 3G11 und dem Hybridom 4G10, mit den ATCC Bezeichnungen HB9847, HB9844, HB9845 bzw. HB9846.

15. Wässrige Zusammensetzung zum Nachweis eines Thrombus bei einem Patienten, wobei die Zusammensetzung ein pharmazeutisch akzeptables Verdünnungsmittel und eine Bildbildungswirksame Menge eines monoklonalen Antikörpers nach Anspruch 3 oder Anspruch 4, verbunden mit einem in vivo Radioisotop-Marker, enthält.

16. Antikörperzusammensetzung, umfassend mindestens zwei monoklonale Antikörper, sezerniert durch ein Hybridom, ausgewählt aus der Gruppe, bestehend aus dem Hybridom 2G5, dem Hybridom 2F10, dem Hybridom 3G11 und dem Hybridom 4G10, mit den ATCC Bezeichnungen HB9847, HB9844, HB9845 bzw. HB9846.

17. Wässrige Zusammensetzung zur Inhibition der Thrombusbildung bei einem Patienten, wobei die Zusammensetzung ein pharmazeutisch akzeptables Verdünnungsmittel und einen monoklonalen Antikörper nach Anspruch 3 oder Anspruch 4 enthält.

18. Wässrige Zusammensetzung zur Inhibition der Blutplättchen-Aggregation bei einem Patienten, wobei die Zusammensetzung ein pharmazeutisch akzeptables Verdünnungsmittel und einen monoklonalen Antikörper nach Anspruch 3 oder Anspruch 4 enthält.

19. Verfahren zur Bildung eines monoklonalen Antikörpermoleküls, das mit einer Rezeptor induzierten Bindungsstelle immunoreagiert, die durch einen ligandenhaltigen Rezeptor-Liganden Komplex exprimiert wird, wobei der Komplex einen Zelloberflächen Rezeptor enthält, der spezifisch an einen Liganden gebunden ist, wobei das Verfahren folgende Schritte umfaßt:
a) Immunisierung eines Säugetiers mit einer Zusammensetzung, die diesen Komplex enthält;
b) Entfernung der Antikörperproduzierenden Zellen aus dem immunisierten Säugetier und Herstellung einer Suspension dieser Zellen;
c) Behandlung der Zellen mit einem Transformationsmittel zur Produktion transformierter Antikörpererzeugender Zellen;
d) Klonierung der Zellen, die in Schritt c) behandelt wurden, mit einer begrenzenden Verdünnung in einem Gewebekulturmedium, das keine nichttransformierten Zellen unterstützen wird, um klonierte Transformanten zu erzeugen;
e) Überprüfung des Gewebekulturmediums der klonierten Transformanten zum Nachweis der Gegenwart sezemierter Antikörpermoleküle die mit einer Rezeptor induzierten Bindestelle immunoreagieren, die auf dem Liganden in dem Rezeptor-Liganden Komplex vorliegt, die aber nicht mit dem Zelloberflächenrezeptor oder dem Liganden immunoreagieren, wenn sich einer von beiden in nicht gebundener Form befindet, wodurch ein klonierter Transformant identifiziert wird, der die Antikörpermoleküle erzeugt;
f) Anzüchtung des identifizierten klonierten Transformanten in einem Gewebekulturmedium unter Bedingungen zu Erzeugung der sezemierten Antikörpermoleküle; und
g) Ernte der sezemierten Antikörpermoleküle aus dem Kulturmedium des Schritts f),
wobei der Ligand Fibrinogen ist und wobei der Rezeptor der GP11b/111a Blutplättchen Glykoprotein Rezeptor ist.

20. In vitro Verfahren zur Überprüfung der Gegenwart eines Rezeptor-Liganden Komplexes in einer vaskulären Flüssigkeitsprobe, wobei der Komplex einen Zelloberflächenrezeptor enthält, der spezifisch an einen Liganden gebunden ist, wobei das Verfahren die folgenden Schritte umfaßt:
a) Bildung einer Immunoreaktionsmischung durch Vermischen einer vaskulären Flüssigkeitsprobe mit einer monoklonalen Antikörperzusammensetzung, die Antikörpermoleküle enthält, die mit einer Rezeptor-induzierten Bindestelle auf dem Liganden in dem Rezeptor-Liganden Komplex immunoreagieren, die jedoch nicht mit dem Zelloberflächenrezeptor oder dem Liganden immunoreagieren, wenn eine von beiden sich in nichtgebundener Form befindet;
b) Erhalten der Mischung über eine ausreichende Zeitspanne, damit die Antikörper mit jeglichen Rezeptor-Liganden Komplexen, die in der Probe vorliegen, immunoreagieren und ein Immunoreaktionsprodukt bilden können; und
c) Nachweis der Gegenwart von jeglichem Immunoreaktionsprodukt, das in Schritt b) gebildet wurde und dadurch der Gegenwart des Komplexes in der Probe,
wobei der Ligand Fibnnogen ist und wobei der Rezeptor das Blutplättchen Glykoprotein GP11b/111a ist.

21. Verfahren nach Anspruch 20, wobei die Antikörpermoleküle die Antikörpermoleküle sind, die durch ein Hybridom erzeugt werden, ausgewählt aus der Gruppe, bestehend aus dem Hybridom 2G5, dem Hybridom 2F10, dem Hybridom 3G11 und dem Hybridom 4G10 mit den ATCC Bezeichnungen HB9847, HB9844, HB9845 bzw. HB9846.

## Revendications

1. Anticorps monoclonal qui immunoréagit avec un site de liaison induit par un récepteur exprimé par un ligand quand ledit ligant est présent dans un complexe récepteur-ligand, mais n'immunoréagit pas avec ledit ligand ou avec ledit récepteur quand soit ledit ligand ou ledit récepteur est libre en solution, le récepteur dudit complexe étant un membre de la super famille de protéines cytoadhésine.

2. Anticorps monoclonal de la revendication 1, où ledit récepteur est GPIIb/IIIa.

3. Anticorps monoclonal de la revendication 2, où ledit ligand est le fibrinogène.

4. Anticorps monoclonal de la revendication 3, où ledit anticorps est sécrété par un hybridome sélectionné dans le groupe consistant en hybridome 2G5, hybridome 2F10, hybridome 3G11 et hybridome 4G10, ayant les désignations ATCC HB9847, HB9844, HB9845 et HB9846, respectivement.

5. Hybridome qui sécrète un anticorps monoclonal selon toute revendication précédente.

6. Hybridome de la revendication 5, où ledit hybridome est sélectionné dans le groupe consistant en hybridome 2G5, hybridome 2F10, hybridome 3G11 et hybridome 4G10, ayant les désignations ATCC HB9847, HB9844, HB9845 et HB9846, respectivement.

7. Composition de culture de cellules comprenant :
a) un hybridome selon la revendication 5 ou la revendication 6 ;
b) des molécules d'anticorps sécrétées par ledit hybridome ; et
c) un milieu de culture pour ledit hybridome.

8. Système de diagnostic en forme de trousse comprenant des anticorps monoclonaux selon l'une quelconque des revendications 1 à 4, lesdites anticorps monoclonaux étant présents dans un emballage en une quantité suffisante pour accomplir au moins un dosage.

9. Système de diagnostic de la revendication 8, où lesdits anticorps monoclonaux sont enchaînés à un moyen indicateur.

10. Système de diagnostic de la revendication 9, où ledit moyen indicateur est emballé séparément desdits anticorps monoclonaux.

11. Système de diagnostic de la revendication 9, où ledit moyen indicateur est enchaîné auxdits anticorps monoclonaux et est un moyen indicateur in vivo.

12. Composition aqueuse pour administration intraveineuse pour disperser un thrombus chez un mammifère, ladite composition contenant un diluant pharmaceutiquement acceptable et une quantité d'un conjugué anticorps monoclonal-enzyme activatrice du plasminogène efficace pour disperser ledit thrombus où ladite portion d'anticorps monoclonal dudit conjugué est un anticorps monoclonal selon la revendication 3.

13. Composition de la revendication 12, où ladite portion d'enzyme activatrice du plasminogène dudit complexe est sélectionnée dans le groupe consistant en streptokinase, urokinase et activateur de plasminogène du tissu.

14. Composition de la revendication 13, où ladite portion d'anticorps monoclonal dudit conjugué est sécrétée par un hybridome sélectionné dans le groupe consistant en hybridome 2G5, hybridome 2F10, hybridome 3G11 et hybridome 4G10, ayant les désignations ATCC HB9847, HB9844, HB9845 et HB9846, respectivement.

15. Composition aqueuse pour détecter un thrombus chez un patient, ladite composition contenant un diluant pharmaceutiquement acceptable et une quantité efficace d'imagerie d'un anticorps monoclonal selon la revendication 3 ou la revendication 4 enchaîné à un marqueur radioisotopique in vivo.

16. Composition d'anticorps comprenant au moins deux anticorps monoclonaux sécrétés par un hybridome sélectionné dans le groupe consistant en hybridome 2G5, hybridome 2F10, hybridome 3G11 et hybridome 4G10, ayant les désignations ATCC HB9847, HB9844, HB9845 et HB9846, respectivement.

17. Composition aqueuse pour inhiber la formation d'un thrombus chez un patient, ladite composition contenant un diluant pharmaceutiquement acceptable et un anticorps monoclonal selon la revendication 3 ou la revendication 4.

18. Composition aqueuse pour l'inhibition de l'agrégation des plaquettes chez un patient, ladite composition contenant un diluant pharmaceutiquement acceptable et un anticorps monoclonal selon la revendication 3 ou la revendication 4.

19. Méthode de formation d'une molécule d'anticorps monoclonal qui immunoréagit avec un site de liaison induit par un récepteur exprimé par un complexe récepteur-ligand contenant un ligand, où ledit complexe contient un récepteur de surface de cellule spécifiquement lié à un ligand, laquelle méthode comprend
a) l'immunisation d'un mammifère avec une composition contenant ledit complexe ;
b) l'enlèvement des cellules productrices d'anticorps dudit mammifère immunisé et la préparation d'une suspension desdites cellules ;
c) le traitement desdites cellules avec un agent transformant pour produire des cellules transformées de production d'anticorps ;
d) le clonage des cellules traitées à l'étape c) par une dilution limite dans un milieu de culture de tissu qui n'entretiendra pas des cellules non transformées, pour produire des transformants clonés ;
5 e) le dosage du milieu de culture de tissu des transformants clonés pour détecter la présence de molécules d'anticorps sécrétées qui immunoréagissent avec un site de liaison induit par un récepteur présent sur le ligand dans ledit complexe récepteur-ligand mais n'immunoréagissent pas avec le récepteur de surface de cellule ou le ligand quand chacun est sous forme non liée pour ainsi identifier un transformant cloné produisant lesdites molécules d'anticorps ;
f) la croissance dudit transformant cloné identifié dans un milieu de culture de tissu dans des conditions pour produire lesdites molécules d'anticorps sécrétées; et
g) la récolte desdites molécules d'anticorps sécrétées du milieu de culture de l'étape f),
où ledit ligand est le fibrinogène et le récepteur est le récepteur de la glycoprotéine des plaquettes GPIIb/IIIa.

20. Méthode in vitro de dosage de la présence d'un complexe récepteur-ligand dans un échantillon de fluide vasculaire où ledit complexe contient un récepteur de surface de cellule spécifiquement lié à un ligand, laquelle méthode comprend les étapes de :
a) former un mélanger d'immunoréaction par mélange d'un échantillon de fluide vasculaire avec une composition d'anticorps monoclonal contenant des molécules d'anticorps qui immunoréagissent avec un site de liaison induit par un récepteur sur le ligand dans ledit complexe ligand-récepteur mais n'immunoréagissent pas avec le récepteur de surface des cellules ou le ligand quand chacun est sous une forme non liée ;
b) maintenir le mélange pendant une période de temps suffisante pour que lesdites anticorps immunoréagissent avec tout complexe récepteur-ligand présent dans l'échantillon et forment un produit d'immunoréaction ; et
c) détecter la présence de tout produit d'immunoréaction formé à l'étape b) et ainsi la présence dudit complexe dans ledit échantillon,
où ledit ligand est le fibrinogène et ledit récepteur est la glycoprotéine des plaquettes GPIIb/IIIa.

21. Méthode de la revendication 20, où lesdites molécules d'anticorps sont les molécules d'anticorps produites par un hybridome sélectionné dans le groupe consistant en hybridome 2G5, hybridome 2F10, hybridome 3G11 et hybridome 4G10, ayant les désignations ATCC HB9847, HB9844, HB9845 et HB9846, respectivement.
